(19) Europäisches Patentamt
European Patent Office
Office européen des brevets

(11) Publication number: 0 268 907 A2

# (12) EUROPEAN PATENT APPLICATION

(21) Application number: **87116450.5**

(22) Date of filing: **06.11.87**

(51) Int. Cl.4: **A61K 31/19**, A61K 31/20, A61K 31/34, A61K 31/35, A61K 31/455

(30) Priority: **19.11.86 US 932954**

(43) Date of publication of application: **01.06.88 Bulletin 88/22**

(84) Designated Contracting States: **DE FR GB IT**

(71) Applicant: **Thornfeldt, Carl Richard**
**1054 N.W. 2nd Avenue**
**Ontario Oregon 97914(US)**

(72) Inventor: **Thornfeldt, Carl Richard**
**1054 N.W. 2nd Avenue**
**Ontario Oregon 97914(US)**

(74) Representative: **Manitz, Gerhart, Dipl.-Phys. Dr. et al**
**MANITZ, FINSTERWALD & ROTERMUND**
**Robert-Koch-Strasse 1**
**D-8000 München 22(DE)**

## (54) Treatment of disease conditions relating to hyperactive organelles.

(57) Disease conditions resulting from the hyperactivity or organelles are treated by administering either percarboxcylic acids or kojic acid esters in an appropriate formulation. The percarboxcylic acids or those of the formula

$$R\text{-}[\text{-}\overset{O}{\overset{\|}{C}}\text{-O-OH}]_n$$

in which R is alkyl, alkenyl, aryl, heteroaryl and substituted derivatives thereof, and n is 1 or 2. The kojic acid esters are those having the formula

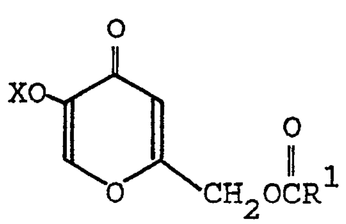

in which X is H or $R^2\text{-}\overset{O}{\overset{\|}{C}}\text{-}$,
and $R^1$ and $R^2$ are either alkyl, alkenyl, or substituted derivatives thereof. These compounds, as well as dicarboxylic acids, also function as carriers for toxic therapeutic agents, to enhance the penetration of such agents into the organelles while inhibiting their toxicity to the host organism.

EP 0 268 907 A2

# TREATMENT OF DISEASE CONDITIONS RELATING TO HYPERACTIVE ORGANELLES

## BACKGROUND OF THE INVENTION

This invention relates to therapies for the treatment of cutaneous diseases and neoplasms associated with hyperactive organelles. Such conditions include a wide range of skin disorders, including inflammation, cellular proliferation and carcinogenesis. In particular, this invention relates to therapeutic compositions for administering to subjects suffering from such conditions.

Therapeutic agents for disease states characterized by hyperactive cellular functions generally achieve their result by the physical or chemical manipulation of intracellular proteins and enzymes. Many protein and enzyme systems are produced or packaged in intracellular organelles including the nucleus, mitochondria, ribosomes, Golgi apparatus, and endoplasmic reticulum. Specialized cells contain unique organelles responsible for the cells' specialized functions. One example is the melanosome, an organelle in the melanocyte.

To reach these protein and enzyme systems, the therapeutic agents must first overcome the barrier of the cell surface which consists of a phospholipid bilayer with scattered suspended glycoproteins. Lipophilic molecules penetrate the lipid bilayer to alter the intracellular enviroment while hydrophilic and very large molecules attach to the glycoprotein structures, producing a change in their architectural configuration and thereby stimulating activation of the adenyl cyclase "second messenger" system. Other molecules are metabolized within the cell wall, releasing active metabolites into the cell interior to alter the intracellular environment.

It is known that the permeability of cell walls and intracellular organelle walls increases in cells with a hyperactive metabolism, such as, for example, those experiencing inflammation, proliferation or carcinogenesis and those controlled or stimulated by a foreign genome. Cell energy expenditure and oxygen uptake is also increased. Many therapeutic agents take advantage of this increased permeability to maximize their effect upon the cell and thus the abnormal state of the organism as a whole. The efficacy of many such agents, however, is limited by their inability to achieve substantial organelle penetration.

## SUMMARY OF THE INVENTION

It has now been discovered that certain chemical species are capable of a high degree of organelle penetration. Accordingly, when such species are administered to a patient suffering from a disease state or abnormality related to hyperactive organelles, such species directly interfere with the abnormal cell processes controlled or performed by the organelles. As a result, the species are unusually effective therapeutic agents for such conditions.

It has further been discovered that these species are capable of targeting the biologically active substances which possess therapeutic properties. Thus, the organelle penetrants of the present invention may be used as carriers for these therapeutic agents, including some that are otherwise toxic, thus carrying the latter into the organelles where they can control, inhibit or eliminate the abnormal cell processes and exhibit the therapeutic action. In essence, the organelle penetrants also display a toxin suppressing capability and thus are useful as carriers of therapeutic agents including toxins such as the A chain of ricin as an example.

Administration of the agents and compositions disclosed herein for both types of therapy may be achieved in a variety of ways. When administration is achieved by topical application, the efficacy of the organelle penetrating agents either alone or accompanied by the therapeutic agents is enhanced by combining these agents with carriers which enhance the penetration of the stratum corneum.

## DETAILED DESCRIPTION OF THE INVENTION

The organelle penetrants of the present invention fall within two groups--percarboxylic acids and kojic acid esters. It has been discovered that the percarboxylic acids are useful for disease conditions other than those of sebaceous glands, and that the kojic acid esters are useful for disease conditions other than those of melanocytes.

The percarboxylic acids are those having the formula

$$R\text{-}[\text{-}\overset{O}{\overset{\|}{C}}\text{-O-OH}]_n$$

in which R is a member selected from the group consisting of $C_2$-$C_{20}$ alkyl, $C_2$-$C_{20}$ alkenyl, aryl, heteroaryl, and derivatives thereof bearing at least one substituent selected from the group consisting of halo, $C_1$-$C_3$ alkyl, hydroxy, $C_1$-$C_3$ alkoxy and carboxy; and n is 1 or 2. Halide salts and anhydrides of these percarboxylic acids are also contemplated as within the scope of the invention.

As used herein, the terms "alkyl," "alkenyl" and "alkoxy" include both straight-chain and branched-chain groups, and "alkenyl" refers to groups containing one or more double bonds. The term "heteroaryl" refers to heterocyclic aromatic groups containing one or more hetero atoms selected from oxygen, nitrogen and sulfur. When two or more hetero atoms are present, they may be the same or different. The terms "aryl" and "heteroaryl" include multicyclic compounds.

Preferred aryl and heteroaryl groups are monocyclic structures with a single hereto atom which is either oxygen or nitrogen. The term "carboxy" refers to the -COOH group.

The percarboxylic acids of the present invention may be prepared according to conventional techniques. For example, carboxylic acid precursors may be converted to the peroxides by treatment with hydrogen peroxide in the presence of sulfuric acid. Examples of precursors for peroxides within the scope of the invention are as follows (with technical names in parentheses):

caprylic (octanoic) acid
capric (decanoic) acid
lauric (dodecanoic) acid
stearic (n-octadecanoic) acid
denzoic acid
3-chlorobenzoic acid
4-chlorobenzoic acid
furan-2-carboxylic acid
maleic (cis-butenedioic) acid
phthalic (ortho-benzenedioic) acid
isophthalic (meta-benzenedioic) acid
terephthalic (para-benzenedioic) acid
adipic (hexanedioic) acid
suberic (octanedioic) acid
azelaic (nonanedioic) acid
sepacic (decanedioic) acid
nicotinic (pyriding-3-carboxylic) acid

The kojic acid esters are those having the formula

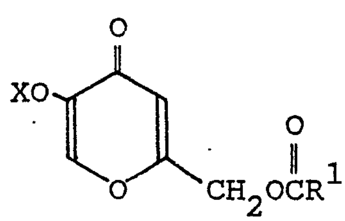

in which X is H or $R^2-\overset{O}{\overset{\|}{C}}-$,
and $R^1$ and $R^2$ are the same or different and are members selected from the group consisting of $C_1$-$C_{30}$ alkyl, $C_2$-$C_{30}$ alkenyl, and derivatives thereof bearing at least one substituent selected from the group consisting hydroxy and carboxy. The considerations mentioned above regarding the meaning and scope of the terms apply here as well. Among the alkyl groups, $C_1$-$C_{20}$ alkyl is preferred.

Preparation of the kojic acid esters may be achieved according to conventional techniques. The monoester compounds may be prepared by reacting kojic acid with an appropriate carboxylic acid in the presence of zinc chloride at an elecated temperature. The diester compounds may be prepared by adding an appropriate carboxylic acid halide to a pyridine solution of kojic acid at a low temperature.

As the listing following the formula indicates, the precursor carboxylic acid may be a mono-, di- or tricarboxylic acid. Examples of such precursors are as follows (with technical names in parentheses):

acetic acid
propionic acid
butyric acid
n-valeric (pentanoic) acid
caproic (hexanoic) acid
caprylic (octanoic) acid
pelargonic (nonanoic) acid
lauric (dodecanoic) acid
myristic (tetradecanoic) acid
palmitic (hexadecanoic) acid
margaric (heptadecanoic) acid
arachidic (eicosanoic) acid
lignoceric (tetraeicosanoic) acid
linoleic (9,12-octadecadienoic) acid
linolenic (9,12,15-octadecatrienoic) acid
maleic (cis-butenedioic) acid
oleic (cis-9-octadecenoic) acid
arachidonic (5,8,11,14-eicosatetraenoic) acid
malonic (methanedicarbonic) acid
succinic (butanedioic) acid
glutaric (pentanedioic) nacid
lactic (alpha-hydroxypropionic) acid
malic (hydroxubutanedioic) acid
tartaric (alpha, beta-dihydroxybutanedioic) acid
citric (2-hydroxy-1,2,3-propanetricarboxylic) acid

The compounds and compositions described above may be administered topically, orally or parenterally in the practice of the invention. The compounds are preferably applied topically in the form of dermatological formulations. These include any of the various known mixtures and combinations which will permit even spreading of the active ingredient over the infected area. Examples include creams, lotions, solutions, ointments, unguents, shampoos, aerosols, gels and pastes.

Additional ingredients may be included to place the compositions in a cosmetically acceptable form. These may include oils, fats, waxes, alcohol, stabilizers, preservatives, surface active agents, dyes, fragrances and conditioners.

Further active agents, notable keratolytic agents, may also be included. Examples of keratolytic agents, are salicylic acid, sulphur and retinoid derivatives. Optimal concentrations will vary among different types of keratolytic agents. Salicylic acid, for example, is preferably used at about 0.5% to about 5.0% while sulphur is preferably used at about 2% to about 10%. Appropriate ranges will be apparent to those skilled in the art.

The concentration of the percarboxylic acid or kojic acid ester in the formulation to be administered is not critical. Concentration will generally vary from about 0.1% to about 40% by weight, depending on the type of formulation and method of administration. Preferred concentrations are from about 10% to about 20% by weight.

Stratum corneum penetrants useful for combination with any of the percarboxylic acids or kojic acid esters described above when applied topically may be selected from those known in the art. The nature and composition of the stratum corneum penetrants is not critical and may vary widely. Examples are:

salicylic acid
dimethyl sulfoxide
propylene glycol
ethyl alcohol
sulfur
AZONE (available from Nelson Research Laboratories, Irvine California)
Transretionic Acid (available from Ortho Pharmazeuticals, Raritan, New Jersey)

The disease states to which the present invention is addressed are those arising from organelle hyperactivity. Examples of such conditions are inflammatory and infectious dermatoses, disorders of keratinization, male pattern baldness, benign and malignant cutaneous tumors and premalignant cutaneous conditions.

As mentioned above, the percarboxylic acids and kojic acid esters described herein as well as individual dicarboxylic acids themselves may be used as toxicity suppressing carriers for toxic therapeutic agents, by carrying such agents into the organelles to exhibit their therapeutic activity. An example of such a biologically active substance is the A chain of ricin. The acids and esters of this invention also may act as carriers of therapeutic agents whose efficacy is inadequate due to low intracellular organelle concentrations. This may be reversed by the carriers with the attached agents specifically penetrating abnormally hyperactive cells and their organelles. An example is methotrexate applied topically in psoriasis.

When the percarboxylic acids or kojic acid esters described herein are combined with the therapeutic agents in therapeutic compositions, the two species may be simply mixed together or they may be bound together as a conjugate by a covalent bond (such as, for example, a disulfide bond), an ionic bond or hydrogen bonds.

The term "therapeutically effective amount" is used herein to denote any amount which will produce a substantial improvement in a disease condition when applied to the affected area repeatedly over a period of time. The amount will vary with the condition being treated, the stage of advancement of the condition, and the type and concentration of the formulation applied. Appropriate amounts in any given instance will by readily apparent to those skilled in the art or capable of determination by routine experimentation.

The foregoing description is offered primarily for purposes of illustration. It will be readily apparent to those skilled in the art that numerous variations in both the formulations and their method of use, not mentioned above, may be made without departing from the spirit and scope of the invention.

## Claims

1. A method for the treatment of a subject suffering from a disease condition resulting from hyperactivity of organelles except those of sebaceous glands, said method comprising administering to said subject a composition comprising a therapeutically effective amount of a compound having the formula

$$R-[-\overset{O}{\overset{\parallel}{C}}-O-OH]_n$$

in which R is a member selected from the group consisting of $C_2$-$C_{20}$ alkyl, $C_2$-$C_{20}$ alkenyl, aryl, heteroaryl, and derivatives thereof bearing at least one substituent selected from the group consisting of halo, $C_1$-$C_3$ alkyl, hydroxy, $C_1$-$C_3$ alkoxy and carboxy; and n is 1 or 2;

or a halide salt or anhydride thereof.

2. A method in accordance with Claim 1 in which R is $C_2$-$C_{20}$ alkyl and n is 1 or 2.

3. A method for administering a biologically active substance having both toxic and therapeutic activity to a subject suffering from a disease controllable thereby, said method comprising administering to said subject a composition comprising:

(a) a therapeutically effective amount of said biologically active substance; and

(b) a toxicity-supressing amount of a compound having the formula

$$R-[-\overset{O}{\overset{\parallel}{C}}-O-OH]_n$$

in which R is a member selected from the group consisting of $C_2$-$C_{20}$ alkyl, $C_2$-$C_{20}$ alkenyl, aryl, heteroaryl, and derivatives thereof bearing at least

one substituent selected from the group consisting of halo, $C_1$-$C_3$ alkyl, hydroxy, $C_1$-$C_3$ alkoxy and carboxy; and n is 1 or 2; or a halide salt or anhydride thereof.

4. A method in accordance with Claim 3 in which R is $C_2$-$C_{20}$ alkyl and n is 1 or 2.

5. A method for the treatment of a subject suffering from a disease condition resulting from hyperactivity of organelles except those of melanoxytes, said method comprising administering to said subject a composition comprising a therapeutically effective amount of a compound having the formula

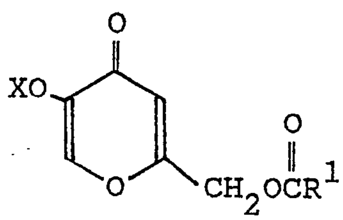

in which X is H or $R^2\text{-}\overset{\text{O}}{\overset{\|}{\text{C}}}\text{-}$,
and $R^1$ and $R^2$ are the same or different and are members selected from the group consisting of $C_1$ -$C_{30}$ alkyl, $C_2$ -$C_{30}$ alkenyl, and derivatives thereof bearing at least one substituent selected from the group consisting of hydroxy and carboxy.

6. A method in accordance with Claim 5
in which X is H or $R^2\text{-}\overset{\text{O}}{\overset{\|}{\text{C}}}\text{-}$,
and $R^1$ and $R^2$ are the same and are members selected from the group consisting of $C_2$-$C_{20}$ alkyl and $C_2$-$C_{20}$ alkenyl.

7. A method for administering a biologically active substance having therapeutic activity to a subject suffering from a disease controllable thereby, said method comprising administering to said subject a composition comprising:

(a) a therapeutically effective amount of said biologically active substance, and

(b) a toxicity-suppressing amount of a compound having the formula

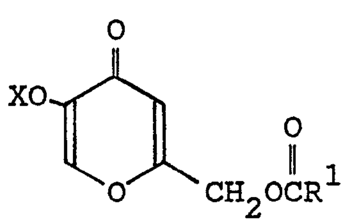

in which X is H or $R^2\text{-}\overset{\text{O}}{\overset{\|}{\text{C}}}\text{-}$,
and $R^1$ and $R^2$ are the same or different and are members selected from the group consisting of $C_1$ -$C_{30}$ alkyl, $C_2$ -$C_{30}$ alkenyl, and derivatives thereof bearing at least one substituent selected from the group consisting of hydroxy and carboxy.

8. A method in accordance with Claim 7
in which X is H or $R^2\text{-}\overset{\text{O}}{\overset{\|}{\text{C}}}\text{-}$,
and $R^1$ and $R^2$ are the same and are members selected from the group consisting of $C_2$-$C_{20}$ alkyl and $C_2$-$C_{20}$ alkenyl.

9. A composition comprising

(a) a therapeutically effective amount of a substance active in the control of a cellular disease condition; and

(b) A cell penetration enhancing amount of a compound having the formula

$$R\text{-}[\text{-}\overset{\text{O}}{\overset{\|}{\text{C}}}\text{-O-OH}]_n$$

in which R is a member selected from the group consisting of $C_2$-$C_{20}$ alkyl, $C_2$-$C_{20}$ alkenyl, aryl, heteroaryl, and derivatives thereof bearing at least one substituent selected from the group consisting of halo, $C_1$-$C_3$ alkyl, hydroxy, $C_1$-$C_3$ alkoxy and carboxy; and n is 1 or 2; or a halide salt or anhydride thereof.

10. A composition comprising

(a) a therapeutically effective amount of a substance active in the control of a cellular disease condition; and

(b) a cell penetration enhancing amount of a compound having the formula

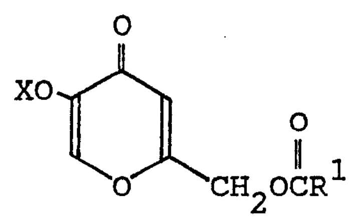

in which X is H or $R^2\text{-}\overset{\text{O}}{\overset{\|}{\text{C}}}\text{-}$,
and $R^1$ and $R^2$ are the same or different and are members selected from the group consisting of $C_1$ -$C_{30}$ alkyl, $C_2$ -$C_{30}$ alkenyl, and derivatives thereof bearing at least one substituent selected from the group consisting of hydroxy and carboxy.